(19) Europäisches Patentamt / European Patent Office / Office européen des brevets

(11) **EP 4 068 149 A1**

(12) **EUROPEAN PATENT APPLICATION**

(43) Date of publication:
**05.10.2022 Bulletin 2022/40**

(21) Application number: **22162334.1**

(22) Date of filing: **15.03.2022**

(51) International Patent Classification (IPC):
**G06F 40/279** (2020.01)    **G06F 40/53** (2020.01)
**G16H 50/00** (2018.01)    **G06N 3/02** (2006.01)
**G06F 40/295** (2020.01)

(52) Cooperative Patent Classification (CPC):
**G06F 40/53; G06F 40/279; G06F 40/295;
G06N 3/02; G16H 50/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2021 CN 202110348806**

(71) Applicant: **Ricoh Company, Ltd.
Tokyo 143-8555 (JP)**

(72) Inventors:
• **Cheng, Rui
  Beijing, 100044 (CN)**
• **Zhang, Yongwei
  Beijing, 100044 (CN)**
• **Jiang, Shanshan
  Beijing, 100044 (CN)**
• **Dong, Bin
  Beijing, 100044 (CN)**

(74) Representative: **Marks & Clerk LLP
15 Fetter Lane
London EC4A 1BW (GB)**

(54) **METHOD AND APPARATUS FOR JOINT-EXTRACTING CLINICAL TEXT ENTITIES AND ATTRIBUTES AND THEIR RELATIONSHIPS**

(57) Disclosed are a method and apparatus for joint-extracting clinical text entities and attributes as well as their relationships. The method includes steps of converting a text to be processed into structured data containing an examination result; inputting the examination result into a pre-trained boundary extraction model to output an extraction result of all data segments in the text; converting a word feature vector of the data segment into a text feature vector and performing positional encoding on the data segment to generate a position feature vector, thereby obtaining a feature vector of the data segment; generating a feature vector of a data segment relationship between any one entity and attribute; and producing a bipartite graph of the data segment and the data segment relationship, and inputting the feature vector of the data segment, the feature vector of the data segment relationship, and a connection relation of the two in the bipartite graph into a pre-trained graph convolutional network model to output class labels.

FIG.8

EP 4 068 149 A1

**Description**

BACKGROUND OF THE DISCLOSURE

1. Field of the Disclosure

The present disclosure relates to the field of data extraction, and particularly, a method and apparatus for joint-extracting clinical text (also called medical examination text) entities and attributes as well as their relationships.

2. Description of the Related Art

In the field of information research, information extraction is a key technology. Information extraction is different from information processing technologies such as information retrieval and the like, and needs to recognize named entities in text and extract the relationships between the named entities. However, in a case of Chinese text, words are flexible and changeable, word formation is complicated, and there are no obvious tokens, thereby making it more difficult to identify Chinese named entities and extract their relationships.

SUMMARY OF THE DISCLOSURE

The present disclosure aims to provide a method and apparatus for joint-extracting clinical text entities and attributes as well as their relationships, by which it is possible to ameliorate the accuracy of the joint extraction result of the clinical text entities and attributes as well as their relationships and improve the efficiency of joint extraction of the clinical text entities and attributes as well as their relationships.

According to a first aspect of the present disclosure, a method of joint-extracting clinical text entities and attributes as well as their relationships is provided that includes steps of:

converting a text to be processed into structured data, wherein, the structured data includes at least one examination result;
inputting the examination result into a pre-trained boundary extraction model to output an extraction result of all data segments in the text to be processed, wherein, the data segments include at least one attribute and entity in the text to be processed;
converting a word feature vector of the data segment into a text feature vector, performing positional encoding on the data segment to generate a position feature vector, and connecting the text feature vector and the position feature vector to form a feature vector of the data segment;
generating a feature vector of a data segment relationship between any one entity and attribute, wherein, the feature vector of the data segment relationship includes the feature vectors of two data segments as well as a previous text feature vector, a middle text feature vector, and a following text feature vector of the two data segments; and
producing a bipartite graph of the data segment and the data segment relationship, and inputting the feature vector of the data segment, the feature vector of the data segment relationship, and a connection relation of the two in the bipartite graph into a pre-trained graph convolutional network model to output a class label of the data segment and a class label of the data segment relationship.

Moreover, in accordance with at least one embodiment, the method further includes a step of training the boundary extraction model. This step includes:

converting at least one training text into structured data, wherein, the structured data involves at least one examination time, examination item, examination result, and a correspondence of the three, and in the training text, the data segment and its class as well as relationships between the data segments and classes of these relationships have been marked in advance; and
training the boundary extraction model by utilizing the training text, wherein, an input of the boundary extraction model is the examination result, an output of the boundary extraction model is an extraction result of all the data segments in the training text, and the data segments involve at least one attribute and entity in the training text.

Furthermore, in accordance with at least one embodiment, a training goal for training the boundary extraction model is to minimize a loss function $L_{span}$ that is calculated by adopting the following equations.

$$P(\hat{t_i}|s) = \text{Softmax}(W_{span}h_i)$$

$$L_{span} = -\frac{1}{|s|}\sum_{i=1}^{|s|}\log P(\hat{t_i} = t_i|s)$$

Here, $\hat{t_i}$ is an i-th label predicted by the boundary extraction model in the examination result; s is the input examination result; $W_{span}$ is a weight parameter of the boundary extraction model that is inclusive of a bi-directional long short-term memory network and a classifier; $h_i$ is a word feature vector representation output from the bi-directional long short-term memory network; $|s|$ is a length of the examination result; and $t_i$ is a real label of an i-th word in the examination result.

Additionally, in accordance with at least one embodiment, the method further includes a step of training the graph convolutional network model. This step includes:

converting a word feature vector of the data segment in the training text into a text feature vector, performing positional encoding on the data segment to generate a position feature vector, and connecting the text feature vector and the position feature vector to form a feature vector of the data segment;

generating a feature vector of the data segment relationship between any one entity and attribute in the training text, wherein, the feature vector of the data segment relationship involves the feature vectors of two data segments as well as a previous text feature vector, a middle text feature vector, and a following text feature vector of the two data segments; and

producing a bipartite graph by using the data segment and the data segment relationship in the training text, and training the graph convolutional network model by utilizing the feature vector of the data segment and the feature vector of the data segment relationship in the training text, wherein, an input of the graph convolutional network model is the feature vector of the data segment, the feature vector of the data segment relationship, and a connection relation of the two in the bipartite graph, and an output of the graph convolutional network model is a class label of the data segment and a class label of the data segment relationship.

Moreover, in accordance with at least one embodiment, a training goal for training a joint extraction model consisting of the graph convolutional network model and the boundary extraction model is to minimize a loss function L that is calculated by adopting the following equations.

$$L = L_{span} + L_{ea} + L_{rel}$$

$$P(\hat{y}|e_i, s) = \text{Softmax}(W_{ea}F_{e_i})$$

$$L_{ea} = -\frac{1}{|\hat{\varepsilon}|} \sum_{e_i \in \hat{\varepsilon}} \log P(\hat{y} = y|e_i, s)$$

$$P(\hat{l}|r_{ij}, s) = \text{Softmax}(W_{rel}F_{r_{ij}})$$

$$L_{rel} = -\sum_{r_{ij}} \frac{\log P(\hat{l} = l|r_{ij}, s)}{|r_{ij}|}$$

Here, $L_{ea}$ is a loss function of a data segment class in the graph convolutional network model; $L_{rel}$ is a loss function of a data segment relationship class in the graph convolutional network model; $\hat{y}$ is a class label of an i-th data segment predicted by the graph convolutional network model; $e_i$ is the i-th data segment; s is the input examination result; $W_{ea}$ and $W_{rel}$ are weight parameters of the graph convolutional network model; $F_{e_i}$ is a feature vector representation of the i-th data segment output from the graph convolutional network model; $|\varepsilon|$ is a total number of all the data segments; $t_i$ is a real label of the i-th data segment; $\hat{l}$ is a class label of a relationship $r_{ij}$ predicted by the graph convolutional network model; the relationship $r_{ij}$ is a relationship between a data segment i and a data segment j; $F_{r_{ij}}$ is a feature vector representation of the relationship $r_{ij}$ output from the graph convolutional network model; $|r_{ij}|$ is a total number of all the relationships; and l is a real label of the relationship $|r_{ij}|$.

In addition, in accordance with at least one embodiment, the step of converting a text to be processed into structured data includes:

extracting at least one examination item in the text to be processed by using a dictionary of examination item terms;

extracting at least one examination time in the text to be processed by utilizing a time extraction rule and template;

extracting, based on at least one text punctuation mark and text distance, at least one examination result in the text to be processed; and

establishing a mapping relation of the examination item, the examination time, and the examination result.

According to a second aspect of the present disclosure, an apparatus for joint-extracting clinical text entities and attributes as well as their relationships is provided that includes:

a conversion module configured to convert a text to be processed into structured data, wherein, the structured data includes at least one examination result;

a boundary extraction module configured to input the examination result into a pre-trained boundary extraction model to output an extraction result of all data segments in the text to be processed, wherein, the data segments include at least one attribute and entity in the text to be processed;

a feature vector generation module configured to convert a word feature vector of the data segment into a text feature vector, perform positional encoding on the data segment to generate a position feature vector, and connect the text feature vector and the position feature vector to form a feature vector of the data segment, and configured to generate a feature vector of a data segment relationship between any one entity and attribute, wherein, the feature vector of the data segment relationship includes the feature vectors of two data segments as well as a previous text feature vector, a middle text feature vector, and a following text feature vector of the two data segments; and

a processing module configured to produce a bipartite graph of the data segment and the data segment relationship, and input the feature vector of the data segment, the feature vector of the data segment relationship, and a connection relation of the two in the bipartite graph into a pre-trained graph convolutional network model to output a class label of the data segment and a class label of the data segment relationship.

Moreover, in accordance with at least one embodiment, the apparatus further includes a first training module configured to train the boundary extraction model. The first training module includes:

a conversion unit configured to convert at least one training text into structured data, wherein, the structured data involves at least one examination time, examination item, examination result, and a correspondence of the three, and in the training text, the data segment and its class as well as relationships between the data segments and classes of these relationships have been marked in advance; and
a first training unit configured to train the boundary extraction model by utilizing the training text, wherein, an input of the boundary extraction model is the examination result, an output of the boundary extraction model is an extraction result of all the data segments in the training text, and the data segments involve at least one attribute and entity in the training text.

Furthermore, in accordance with at least one embodiment, a training goal for training the boundary extraction model is to minimize a loss function $L_{span}$ that is calculated by adopting the following equations.

$$P(\hat{t_i}|s) = \text{Softmax}(W_{span}h_i)$$

$$L_{span} = -\frac{1}{|s|}\sum_{i=1}^{|s|} \log P(\hat{t_i} = t_i|s)$$

Here, $\hat{t_i}$ is an i-th label predicted by the boundary extraction model in the examination result; s is the input examination result; $W_{span}$ is a weight parameter of the boundary extraction model that is inclusive of a bidirectional long short-term memory network and a classifier; $h_i$ is a word feature vector representation output from the bi-directional long short-term memory network; |s| is a length of the examination result; and $t_i$ is a real label of an i-th word in the examination result.

Additionally, in accordance with at least one embodiment, the apparatus further includes a second training module configured to train the graph convolutional network model. The second training module includes:

a feature vector generation unit configured to convert a word feature vector of the data segment in the training text into a text feature vector, perform positional encoding on the data segment to generate a position feature vector, and connect the text feature vector and the position feature vector to form a feature vector of the data segment, and configured to generate a feature vector of the data segment relationship between any one entity and attribute in the training text, wherein, the feature vector of the data segment relationship involves the feature vectors of two data segments as well as a previous text feature vector, a middle text feature vector, and a following text feature vector of the two data segments; and
a second training unit configured to produce a bipartite graph by using the data segment and the data segment relationship in the training text, and train the graph convolutional network model by utilizing the feature vector of the data segment and the feature vector of the data segment relationship in the training text, wherein, an input of the graph convolutional network model is the feature vector of the data segment, the feature vector of the data segment relationship, and a connection relation of the two in the bipartite graph, and an output of the graph convolutional network model is a class label of the data segment and a class label of the data segment relationship.

Moreover, in accordance with at least one embodiment, a training goal for training a joint extraction model consisting of the graph convolutional network model and the boundary extraction model is to minimize a loss function L that is calculated by adopting the following equations.

$$L = L_{span} + L_{ea} + L_{rel}$$

$$P(\hat{y}|e_i, s) = \text{Softmax}(W_{ea}F_{e_i})$$

$$L_{ea} = -\frac{1}{|\hat{\varepsilon}|}\sum_{e_i \in \hat{\varepsilon}} \log P(\hat{y} = y|e_i, s)$$

$$P(\hat{l}|r_{ij}, s) = \text{Softmax}(W_{rel}F_{r_{ij}})$$

$$L_{rel} = -\sum_{r_{ij}} \frac{\log P(\hat{l} = l | r_{ij}, s)}{|r_{ij}|}$$

Here, $L_{ea}$ is a loss function of a data segment class in the graph convolutional network model; $L_{rel}$ is a loss function of a data segment relationship class in the graph convolutional network model; $\hat{y}$ is a class label of an i-th data segment predicted by the graph convolutional network model; $e_i$ is the i-th data segment; s is the input examination result; $W_{ea}$ and $W_{rel}$ are weight parameters of the graph convolutional network model; $F_{e_i}$ is a feature vector representation of the i-th data segment output from the graph convolutional network model; $|\hat{\varepsilon}|$ is a total number of all the data segments; $t_i$ is a real label of the i-th data segment; $\hat{l}$ is a class label of a relationship $r_{ij}$ predicted by the graph convolutional network model; the relationship $r_{ij}$ is a relationship between a data segment i and a data segment j; $F_{rij}$ is a feature vector representation of the relationship $r_{ij}$ output from the graph convolutional network model; $|r_{ij}|$ is a total number of all the relationships; and l is a real label of the relationship $|r_{ij}|$.

In addition, in accordance with at least one embodiment, the conversion module includes:

a first extraction unit configured to extract at least one examination item in the text to be processed by using a dictionary of examination item terms;
a second extraction unit configured to extract at least one examination time in the text to be processed by utilizing a time extraction rule and template;
a third extraction unit configured to extract, based on at least one text punctuation mark and text distance, at least one examination result in the text to be processed; and
a mapping relation establishment unit configured to establish a mapping relation of the examination item, the examination time, and the examination result.

According to a third aspect of the present disclosure, an apparatus for joint-extracting clinical text entities and attributes as well as their relationships is provided that includes:

a storage storing computer-executable instructions; and
a processor coupled to the storage,
wherein, the computer-executable instructions, when executed, cause the processor to conduct the method according to the first aspect of the present disclosure.

According to a fourth aspect of the present disclosure, a non-transitory computer-readable medium storing computer-executable instructions for execution by a processor is provided. The computer-executable instructions, when executed, cause the processor to perform the method according to the first aspect of the present disclosure.
According to a fifth aspect of the present disclosure, a computer program for causing a computer to execute the method according to the first aspect of the present disclosure is provided.
Compared to the prior art, by making use of the method and apparatus for joint-extracting clinical text entities and attributes as well as their relationships, according to the embodiments of the present disclosure, entity and attribute extraction can be performed after a text to be processed is converted into structured data, thereby being able to reduce data noise, facilitate feature extraction, and improve computing performance. In addition, by combining the text feature vector and the position feature vector of a data segment in the text to be processed as the feature vector of the data segment, it is possible to better express the entities and attributes as well as their relationships. The technical solution in accordance with the embodiments is efficient and easy to implement, and can ameliorate the accuracy of the joint extraction result of the clinical text entities and attributes as well as their relationships and improve the efficiency of joint extraction of the clinical text entities and attributes as well as their relationships.

BRIEF DESCRIPTION OF THE DRAWINGS

FIG. 1 is a flowchart of a method of joint-extracting clinical text entities and attributes as well as their relationships, according to a first embodiment of the present disclosure;
FIG. 2 illustrates an exemplary clinical text in Chinese;
FIG. 3 shows exemplary structured data in Chinese;
FIG. 4 presents an example of extracting data segments;
FIG. 5 shows an example of performing positional encoding;
FIG. 6 illustrates an example of generating span nodes (here, a span means a data segment (also called first data));
FIG. 7 presents an example of generating span relationship nodes;
FIG. 8 is a block diagram of an apparatus for joint-extracting clinical text entities and attributes as well as their relationships, according to a second embodiment of the present disclosure; and
FIG. 9 is a block diagram of another apparatus for joint-extracting clinical text entities and attributes as well as their relationships, according to a third embodiment of the present disclosure.

## DETAILED DESCRIPTION OF THE PREFERRED EMBODIMENTS

In order to let a person skilled in the art better understand the present disclosure, hereinafter, the embodiments of the present disclosure are concretely described with reference to the drawings. However, it should be noted that the same symbols, that are in the specification and the drawings, stand for composition elements having basically the same function and structure, and the repetition of the explanations to the composition elements is omitted.

In the embodiments of the present disclosure, a method and apparatus for joint-extracting clinical text entities and attributes as well as their relationships are provided by which it is possible to ameliorate the accuracy of the joint extraction result of the clinical text entities and attributes as well as their relationships and improve the efficiency of joint extraction of the clinical text entities and attributes as well as their relationships.

<First Embodiment

A method of joint-extracting clinical text entities and attributes as well as their relationships is provided in this embodiment.

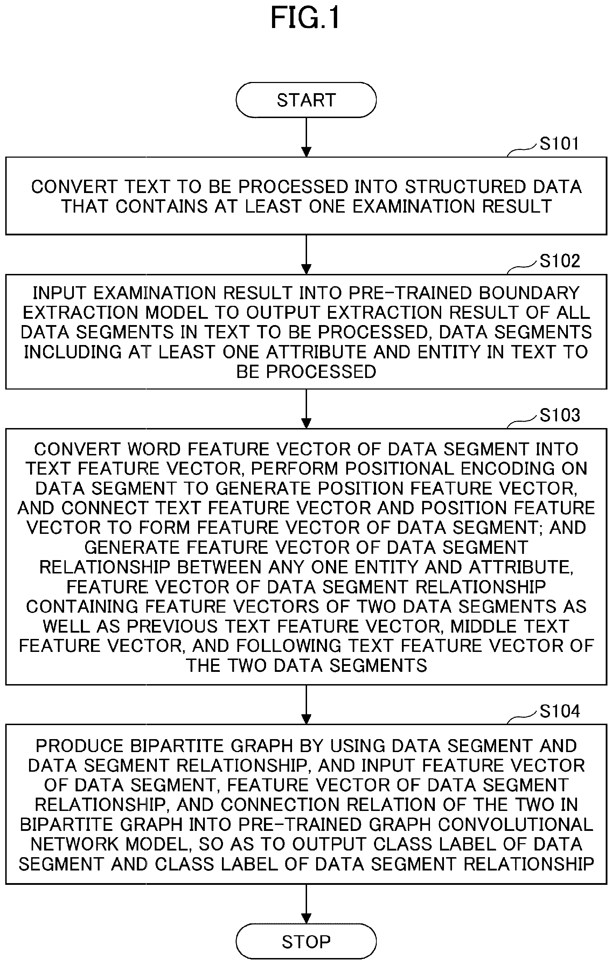

FIG. 1 is a flowchart of a method of joint-extracting clinical text entities and attributes as well as their relationships in accordance with this embodiment. As shown in FIG. 1, the method includes STEPS S101 to S104.

STEP S101 is converting a text to be processed (also called a text waiting for processing) into structured data that contains at least one examination result.

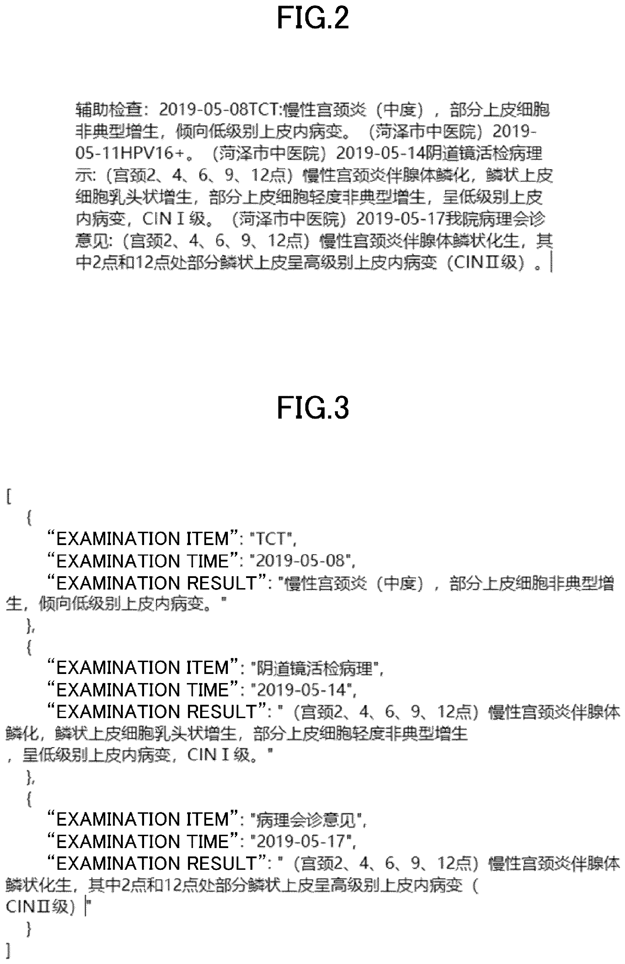

In this embodiment, the text to be processed may be a clinical text (also called a medical examination text). FIG. 2 illustrates an exemplary clinical text in Chinese. As presented in FIG. 2, each sentence in the clinical text is relatively long so that it is not conducive to entity and attribute extraction. As such, it is necessary to convert the clinical text into structured data with a predetermined format.

Furthermore, in this embodiment, a dictionary of examination item terms can be utilized to extract at least one examination item in the text to be processed. The terms in the dictionary may be collected and organized from a large amount of medical data (including medical treatment guidelines, medical records, etc.) by rules and manual labor, and then, sorted in descending order of term length. It is possible to extract, according to a longest matching and extraction principle, at least one examination item in a clinical text, such as TCT (Thinprep Cytology Test), colposcopy biopsy pathology, etc.

Moreover, in this embodiment, a time extraction rule and template can be utilized to extract at least one examination time in the text to be processed. After counting the possible appearance forms of the examination times in a large number of medical examination texts, it is found that an examination time and an examination result have a relative positional relation, i.e., the examination time is either before the examination result (positive order) or after the examination result (reverse order). Thus, it is possible to adopt a positive order based time extraction approach or a reverse order based time extraction approach to extract at least one examination time in the text to be processed.

Additionally, in this embodiment, at least one examination result in the text to be processed can be extracted on the basis of at least one text punctuation mark and text distance. Through the investigation of a large number of medical examination texts, it is found that an examination time, an examination item, and an examination result in a medical examination text are usually close together, and in general, different examination descriptions in a medical examination text are separated by punctuation marks such as periods, semicolons, exclamation marks, question marks, and so on. Hence, it is possible to extract, based on at least one text punctuation mark and text distance, at least one examination result in the text to be processed.

Subsequently, the examination item, examination time, and examination result extracted can be mapped mutually so as to establish a mapping relation of the three, thereby forming the structured data.

In a specific example, the text to be processed is the clinical text shown in FIG. 2, and the converted structured data is illustrated in FIG. 3.

STEP S102 is inputting the examination result into a pre-trained boundary extraction model to output an extraction result of all the data segments in the text to be processed. Here, the data segments include at least one attribute and entity in the text to be processed.

In this embodiment, it is necessary to pre-train the boundary extraction model. The input of the boundary extraction model is at least one examination result in a clinical text, and the output is an extraction result of all the data segments (here, a data segment (i.e., first data) is indicated by a span for short, and the data segments involve at least one attribute and entity) in the clinical text.

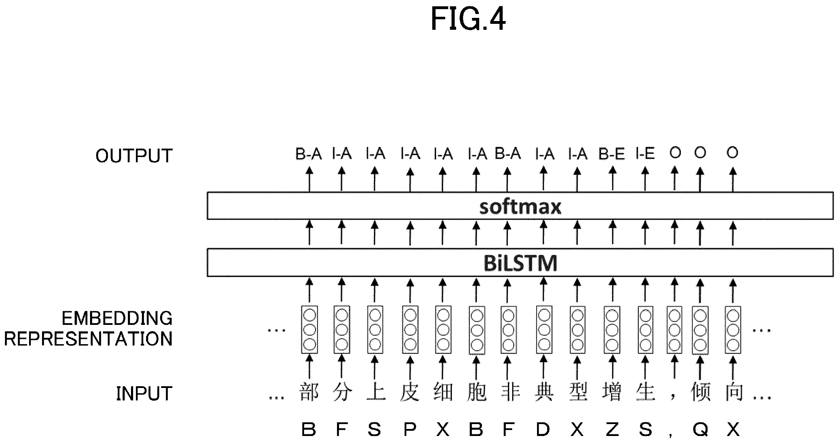

Furthermore, in this embodiment, the boundary extraction model may adopt any entity extraction model. In a specific example, as shown in FIG. 4, the boundary extraction model includes a BiLSTM (bi-directional long short-term memory) model (network) and a classifier softmax.

When training the boundary extraction model, first, at least one training text is converted into structed data that contains at least one examination time, examination item, and examination result as well as the correspondence of the three. Here, in the training text, data segments and their types (classes) as well as the relationships between the data segments and the types of the relationships have been marked in advance. Next, the training text is utilized to train the boundary extraction model. Here, the input of the boundary extraction model is the examination result, and the output of the boundary extraction model is an extraction result of all the data segments involving at least one attribute and entitie in the training text.

As shown in FIG. 4, in a specific example, the input of the boundary extraction model is "Bu Fen Shang Pi Xi Bao Fei Dian Xing Zeng Sheng, Qing Xiang" which are the Chinese pronunciations of a part of Chinese characters contained in the clinal text presented in FIG. 2, and are indicated by "B F S P X B F D X Z S, Q X" in FIG. 4 for the sake of convenience. The output of the boundary extraction model is an extraction result of spans. The concrete form of the extraction result of spans is a sequence of labels in which "B" refers to the beginning position of a span, "I" is indicative of belonging to a span, "O" is indicative of not belonging to a span, "A" indicates that the span is an attribute, and "E" indicates that the span is an entity.

The training goal for training the boundary extraction model is to minimize a loss function $L_{span}$ that may be calculated by adopting the following equations.

$$P(\widehat{t_i}|s) = \text{Softmax}(W_{span}h_i)$$

$$L_{span} = -\frac{1}{|s|}\sum_{i=1}^{|s|}\log P(\widehat{t_i} = t_i|s)$$

Here, $\widehat{t}_i$ is an i-th label predicted by the boundary extraction model in the examination result; s is the input examination result; $W_{span}$ is a weight parameter of the boundary extraction model that is inclusive of a bidirectional long short-term memory (BiLSTM) network and a classifier; $h_i$ is a word feature vector representation output from the BiLSTM network; |s| is the length of the examination result; and $t_i$ is the real label of an i-th word in the examination result.

After training the boundary extraction model, by inputting the examination result obtained in STEP S101 into the boundary extraction model, it is possible to acquire spans. However, in the step, only the spans are output; that is, their types are not given. In this embodiment, the types of the spans are inclusive of entities and attributes. The entities mainly refer to diseases, lesions, symptoms, etc., for example, "Z S" (whose meaning in English is "proliferation") in FIG. 4. The attributes are indicative of the terms describing the entities, such as the regions of lesions (e.g., "B F S P X B" (whose meaning in English is "some epithelial cells" ) in FIG. 4), the nature of the lesions (e.g., "F D X" (whose meaning in English is "atypical") in FIG. 4), etc.

STEP S103 is converting the word feature vector of the data segment into a text feature vector, performing positional encoding on the data segment to generate a position feature vector, and connecting the text feature vector and the position feature vector to form the feature vector of the data segment; and generating the feature vector of the data segment relationship between any one entity and attribute. The feature vector of the data segment relationship contains the feature vectors of two data segments as well as the previous text feature vector, the middle text feature vector, and the following text feature vector of the two data segments.

Here, the data segment may also be called a span node, and the data segment relationship may also be called a span relationship node (i.e., an inter-span relationship node). The span node and the span relationship node are nodes to be subsequently input into the graph convolutional network model. The data segment relationship is the relationship between two data segments. The two data segments include any one entity, and further include an attribute.

By letting the span boundary result obtained and the text vector feature representation eventually output from the BiLSTM model in STEP S102 pass through a dimensional transformation model, it is possible to convert a span (represented by multiple word feature vectors) into a feature vector representation to stand for the text feature vector of the span. Here, the dimensional transformation model may be a CNN (Convolutional Neural Network) + pooling (pooling layer(s)) model or any other suitable dimensional transformation model.

In addition, a positional encoding mechanism can be adopted to generate a span position feature vector representation, and then, the span text feature vector representation and the span position feature vector representation can be connected (combined) to express the feature vector of a span(s).

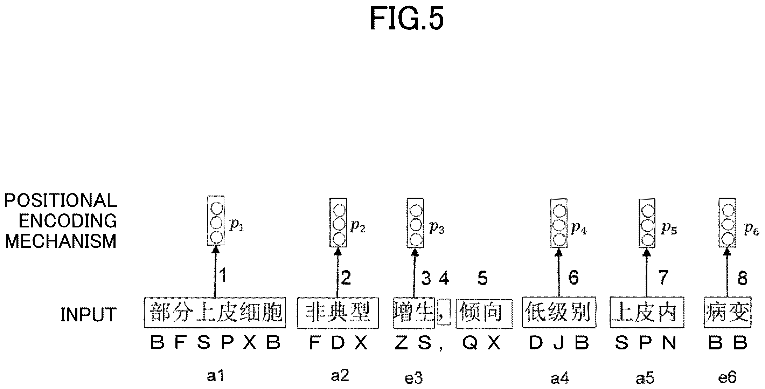

The positional encoding mechanism is shown in FIG. 5. The spans (i.e., entities and attributes (e.g., a1, a2, e3, a4, a5, and e6 in FIG. 5)) obtained in STEP S102 are called useful spans. Each of the useful spans occupies a position. A punctuation mark in the related examination result is regarded as a useless span (e.g., the comma "," in FIG. 5), and the text between two useful spans or between a useful span and a punctuation mark is also regarded as a useless span (e.g., the text "Q X" (whose meaning in English is "tend to") in FIG. 5). Each of the useless spans also occupies a position. Finally, all the spans (including the useful and useless spans) are numbered from left to right, so as to attain a sequence of numbers (e.g., 1, 2, 3, 4, 5, 6, 7, and 8 in FIG. 5). Such a sequence of numbers may express the position or distance relationship of the useful spans, which is very effective information for the prediction of the final span type and span relationship type.

The sequence of numbers, i.e., the position numbers of all the spans (e.g., 1, 2, 3, 4, 5, 6, 7, and 8 in FIG. 5) can be converted, through the dimensional transformation model, into a feature vector representation of the useful spans, i.e., a span position feature vector $p_i$ (e.g., $p_1$, $p_2$, $p_3$, $p_4$, $p_3$, and $p_6$ in FIG. 5).

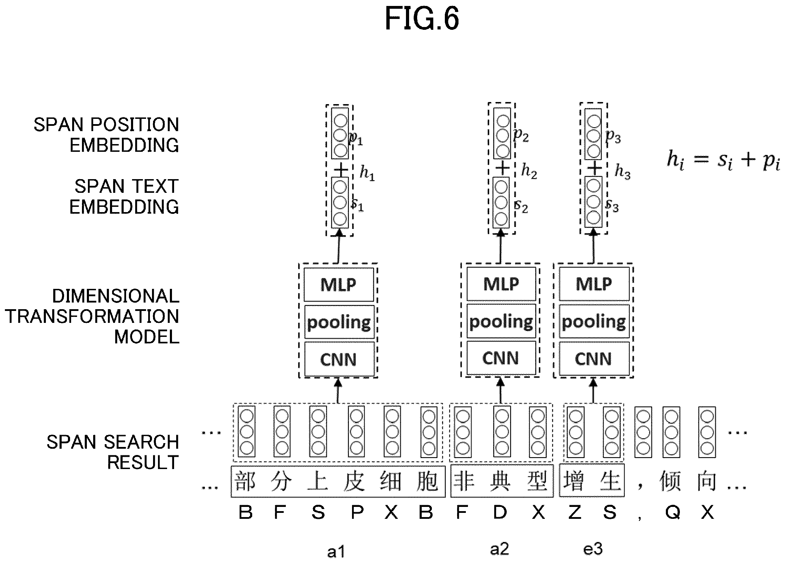

As presented in FIG. 6, the position feature vector $p_i$ and the text feature vector $s_i$ of the useful spans can be connected to form a feature vector $h_i$ of the useful spans; that is, the span nodes can be generated as follows.

$$h_i = s_i + p_i$$

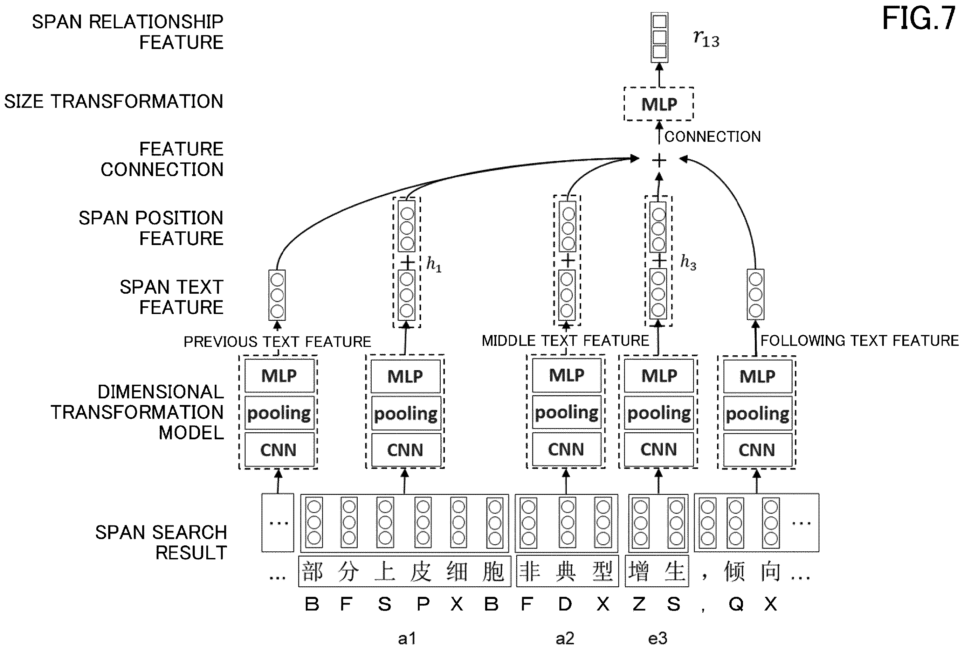

Here, the span (i.e., data segment) relationship refers to the relationship between an entity and an attribute. As illustrated in FIG. 7, a relationship $r_{13}$ is formed between a1 and e3. The feature of a span relationship node mainly includes two parts, namely, (1) an entity span (i.e., e3) feature and an attribute span (i.e., a1) feature that form the span relationship; and (2) a context feature consisting of three parts (also called a previous text feature, a middle text feature, and a following text feature) that exist before, between, and after the two spans, respectively.

For the entity span and the attribute span that form the span relationship, respective feature vectors are generated.

Additionally, for the three parts, i.e., the previous text feature, the middle text feature, and the following text feature forming the context feature, they are converted, by using the dimensional transformation model in the same way as the span feature vector is generated, to produce a previous text feature vector, a middle text feature vector, and a following text feature vector, respectively. Subsequently, these five feature vectors, i.e., the entity span feature vector, the attribute span feature vector, the previous text feature vector, the middle text feature vector, and the following text feature vector are connected to form, after size transformation, a final span relationship feature vector; that is, a span relationship node is generated, as shown in FIG. 7.

Here, the size transformation is to ensure that the size of the final span relationship feature vector matches the size of the span feature vector. The size transformation may be achieved by an MLP (Multilayer Neural Network) model.

Referring again to FIG. 1; STEP S104 is producing a bipartite graph by using the data segment and the data segment relationship, and inputting the feature vector of the data segment, the feature vector of the data segment relationship, and the connection relation of the two in the bipartite graph into a pre-trained graph convolutional network model, so as to output the class label of the data segment and the class label of the data segment relationship.

In this example, it is necessary to pre-train the graph convolutional network model. The input of the graph convolutional network model is the span node, the span relationship node, and the structural relation between the two (i.e., the connection relation of the span node and the span relationship node in the bipartite graph), namely, the feature vector of the data segment, the feature vector of the data segment relationship, and the structural relation of the two. The output of the graph convolutional network model is the types of the said two nodes, i.e., the class labels of the data segment and the data segment relationship.

By letting the span relationship node be connected to the two span nodes forming the span relationship node, respectively, it is possible to produce the bipartite graph. For example, as illustrated in FIG. 7, a bipartite graph can be generated by utilizing the span nodes $h_1$ and $h_3$ as well as the span relationship node $r_{13}$. In this bipartite graph, the span relationship node $r_{13}$ is connected to the span nodes $h_1$ and $h_3$, respectively. Each of the connection relation between the span relationship node $r_{13}$ and the span nodes $h_1$ and the connection relation between the span relationship node $r_{13}$ and the span nodes $h_3$ is the connection relation between the span node and the span relationship node, namely, the structural relation between the feature vector of the data segment and the feature vector of the data segment relationship.

In this embodiment, the graph convolutional network model includes a GCNN (Graph Convolutional Neural Network) model and a classifier softmax.

The step of training the graph convolutional network model is inclusive of:

converting the word feature vector of the data segment in the training text into a text feature vector, performing positional encoding on the data segment to generate a position feature vector, and connecting the text feature vector and the position feature vector to form the feature vector of the data segment;

generating the feature vector of the data segment relationship between any one entity and attribute in the training text, wherein, the feature vector of the data segment relationship includes the feature vectors of two data segments as well as the previous text feature vector, the middle text feature vector, and the following text feature vector of the two data segments; and

producing a bipartite graph by using the data segment and the data segment relationship in the training text, and training the graph convolutional network model by utilizing the feature vector of the data segment and the feature vector of the data segment relationship, wherein, the input of the graph convolutional network model is the feature vector of the data segment, the feature vector of the data segment relationship, and the connection relation of the two in the bipartite graph, and the output of the graph convolutional network model is the class label of the data segment and the class label of the data segment relationship.

The training goal for training a joint extraction model consisting of the graph convolutional network model and the boundary extraction model is to minimize a loss function L that may be calculated by adopting the following equations.

$$L = L_{span} + L_{ea} + L_{rel}$$

$$P(\hat{y}|e_i, s) = \text{Softmax}(W_{ea}F_{e_i})$$

$$L_{ea} = -\frac{1}{|\hat{\varepsilon}|}\sum_{e_i \in \hat{\varepsilon}} \log P(\hat{y} = y|e_i, s)$$

$$P(\hat{l}|r_{ij}, s) = \text{Softmax}(W_{rel}F_{r_{ij}})$$

$$L_{rel} = -\sum_{r_{ij}} \frac{\log P(\hat{l} = l|r_{ij}, s)}{|r_{ij}|}$$

Here, $L_{ea}$ is a span node classification loss function, i.e., a loss function of the data segment class in the graph convolutional network model; $L_{rel}$ is a span relationship node classification loss function, i.e., a loss function of a data segment relationship class in the graph convolutional network model; $\hat{y}$ is the class label of an i-th data segment predicted by the graph convolutional network model; $e_i$ is the i-th data segment; s is the input examination result; $W_{ea}$ and $W_{rel}$ are the weight parameters of the graph convolutional network model; $F_{e_i}$ is the feature vector representation of the i-th data segment output from the graph convolutional network model; $|\hat{\varepsilon}|$ is the total number of all the data segments; $t_i$ is the real label of the i-th data segment; 1 is the class label of a relationship $r_{ij}$ predicted by the graph convolutional network model; the relationship $r_{ij}$ is the relationship between a data segment i and a data segment j; $F_{r_{ij}}$ is the feature vector representation of the relationship $r_{ij}$ output from the graph convolutional network model; $|r_{ij}|$ is the total number of all the relationships; and 1 is the real label of the relationship $|r_{ij}|$.

The training goal of the entire joint extraction model is to obtain the minimum total loss function based score (i.e., the minimum overall cost). Subsequently, by storing the parameters of the model, it is possible to use the model to conduct prediction. Specifically, by inputting the span node and the span relationship node acquired in STEP S103 into the trained graph convolutional network model and utilizing a classifier (e.g., softmax) to classify the relevant features, it is possible to output the type of each node.

In this embodiment, because the span nodes and the span relationship nodes are employed to obtain the types of the nodes, and since the relationships between the nodes are considered, the accuracy of the joint extraction result of the clinical text entities and attributes as well as their relationships can be improved, and the efficiency of joint extraction of the clinical text entities and attributes as well as their relationships can also be ameliorated.

Furthermore, in this embodiment, the types of the entity nodes include diseases, symptoms, lesions, etc., the types of the attribute nodes include regions, nature of lesions, etc., and the types of relationship nodes are formed by combining the types of the entity and attribute nodes. As a result, the types of the nodes can be adjusted according to practical applications.

Compared to the prior art, by utilizing the method of joint-extracting clinical text entities and attributes as well as their relationships, according to this embodiment, entity and attribute extraction can be performed after a text to be processed is converted into structured data, thereby being able to reduce data noise, facilitate feature extraction, and improve computing performance. In addition, by combining the text feature vector and the position feature vector of a data segment in the text to be processed as the feature vector of the data segment, it is possible to better express the entities and attributes as well as their relationships. The technical solution in accordance with this embodiment is efficient and easy to implement, and can ameliorate the accuracy of the joint extraction result of the clinical text entities and attributes as well as their relationships and improve the efficiency of joint extraction of the clinical text entities and attributes as well as their relationships.

<Second Embodiment

In this embodiment, an apparatus for joint-extracting clinical text entities and attributes as well as their relationships is provided.

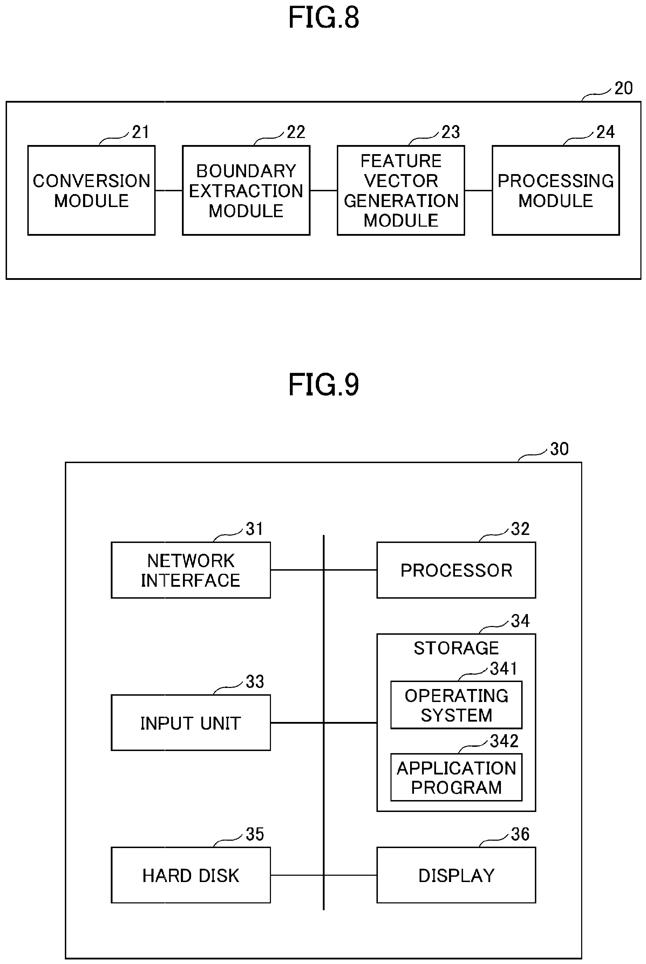

FIG. 8 is a block diagram of an apparatus 20 for joint-extracting clinical text entities and attributes as well as their relationships, according to this embodiment.

As illustrated in FIG. 8, the apparatus 20 may contain a conversion module 21, a boundary extraction module 22, a feature vector generation module 23, and a processing module 24. Of course, the apparatus 20 may also include other modules as needed.

The apparatus 20 can be configured to perform the method of joint-extracting clinical text entities and attributes as well as their relationships, in accordance with the first embodiment. Specifically, the conversion module 21, the boundary extraction module 22, the feature vector generation module 23, and the processing module 24 may be configured to conduct STEPS S101 to S104 of FIG. 1, respectively.

Here, it should be mentioned that because STEPS S101 to S104 of FIG. 1 have been concretely described in the first embodiment, the details of them are omitted in this embodiment for the sake of convenience.

Moreover, the conversion module 21 may involve a first extraction unit, a second extraction unit, a third extraction unit, and a mapping relation establishment unit (not shown in the drawings) respectively configured to extract at least one examination item, examination time, and examination result in a text waiting for processing and establish a mapping relation of the three. For more information, it is possible to see the description related to STEP S101 in the first embodiment.

Furthermore, the apparatus 20 may further include a first training module and a second training module (not presented in the drawings). The first training module may involve a conversion unit and a first training unit respectively configured to convert a training text into structured data and train a boundary extraction model by using the structured data of the training text. The second training module may involve a feature vector generation unit and a second training unit respectively configured to generate the feature vector of at least one data segment and the feature vector of at least one data segment relationship in a training text and train a graph conventional network model by utilizing the feature vector of the at least one data segment and the feature vector of the at least one data segment relationship in the training text. For more information, it is also possible to see the descriptions relating to the corresponding steps in the first embodiment.

Compared to the prior art, by taking advantage of the apparatus for joint-extracting clinical text entities and attributes as well as their relationships, according to this embodiment, entity and attribute extraction can be performed after a text to be processed is converted into structured data, thereby being able to reduce data noise, facilitate feature extraction, and improve computing performance. In addition, by combining the text feature vector and position feature vector of a data segment in the text to be processed as the feature vector of the data segment, it is possible to better express the entities and attributes as well as their relationships. The technical solution in accordance with this embodiment is efficient and easy to implement, and can ameliorate the accuracy of the joint extraction result of the clinical text entities and attributes as well as their relationships and improve the efficiency of joint extraction of the clinical text entities and attributes as well as their relationships.

<Third Embodiment

Another apparatus for joint-extracting clinical text entities and attributes as well as their relationships is provided in this embodiment.

FIG. 9 is a block diagram of an apparatus 30 for joint-extracting clinical text entities and attributes as well as their relationships, according to this embodiment.

As presented in FIG. 9, the apparatus 30 is inclusive of a network interface 31, a processor 32, an input unit 33, a storage 34 including an operating system 341 and an application program 342, a hard disk 35, and a display 36 which are connected by a bus.

The network interface 31 may be configured to connect to a network such as the Internet, a local area network (LAN), or the like. The processor 32 may be used to execute a computer program, for example, the application program 342 stored in the storage 34, so as to fulfill the method according to the first embodiment. The input unit 33 may be configured to let a user input various instructions, which may be a keyboard or a touch panel, for instance. The storage 34 may be utilized to store requisite computer programs and data as well as the intermediate results generated when the processor 32 conducts the application program 342, for example. The hard disk 35 may be employed to store any information or data necessary to achieve the method according to the first embodiments, for instance. The display 36 may be used to display the results acquired when executing the application program 342 by the processor 32, for example.

Additionally, a computer-executable program and a non-transitory computer-readable medium are further provided. The computer-executable program may cause a computer to perform the method according to the first embodiment. The non-transitory computer-readable medium may store computer-executable instructions (i.e., the computer-executable program) for execution by a computer involving a processor. The computer-executable instructions may, when executed by the processor, render the processor to conduct the method in accordance with the first embodiment.

Here, it should be noted that because the steps involved in the method according to the first embodiment have been minutely set forth above, the descriptions of the steps are omitted in this embodiment for the sake of convenience.

In addition, the above embodiments are just exemplary ones, and the specific structure and operation of them may not be used for limiting the present disclosure.

Furthermore, the embodiments of the present disclosure may be implemented in any convenient form, for example, using dedicated hardware or a mixture of dedicated hardware and software. The embodiments of the present disclosure may be implemented as computer software implemented by one or more networked processing apparatuses. The network may comprise any conventional terrestrial or wireless communications network, such as the Internet. The processing apparatuses may comprise any suitably programmed apparatuses such as a generalpurpose computer, a personal digital assistant, a mobile telephone (such as a WAP or 3G, 4G, or 5G-compliant phone), and so on. Since the embodiments of the present disclosure may be implemented as software, each and every aspect of the present disclosure thus encompasses computer software implementable on a programmable device.

The computer software may be provided to the programmable device using any storage medium for storing processor-readable code such as a floppy disk, a hard disk, a CD ROM, a magnetic tape device, or a solid state memory device.

The hardware platform includes any desired hardware resources including, for example, a central processing unit (CPU), a random access memory (RAM), and a hard disk drive (HDD). The CPU may include processors of any desired type and number. The RAM may include any desired volatile or nonvolatile memory. The HDD may include any desired nonvolatile memory capable of storing a large amount of data. The hardware resources may further include an input device, an output device, and a network device in accordance with the type of the apparatus. The HDD may be provided external to the apparatus as long as the HDD is accessible from the apparatus. In this case, the CPU, for example, the cache memory of the CPU, and the RAM may operate as a physical memory or a primary memory of the apparatus, while the HDD may operate as a secondary memory of the apparatus.

While the present disclosure is described with reference to the specific embodiments chosen for purpose of illustration, it should be apparent that the present disclosure is not limited to these embodiments, but numerous modifications could be made thereto by a person skilled in the art without departing from the basic concept and technical scope of the present disclosure.

The present application is based on and claims the benefit of priority of Chinese Patent Application No. 202110348806.X filed on March 31, 2021, the entire contents of which are hereby incorporated by reference.

## Claims

1. A method of joint-extracting clinical text entities and attributes as well as their relationships, comprising:

   a step of converting a text to be processed into structured data, wherein, the structured data includes at least one examination result;
   a step of inputting the examination result into a pre-trained boundary extraction model to output an extraction result of all data segments in the text to be processed, wherein, the data segments include at least one attribute and entity in the text to be processed;
   a step of converting a word feature vector of the data segment into a text feature vector, performing positional encoding on the data segment to generate a position feature vector, and connecting the text feature vector and the position feature vector to form a feature vector of the data segment;
   a step of generating a feature vector of a data segment relationship between any one entity and attribute, wherein, the feature vector of the data segment relationship includes the feature vectors of two data segments as well as a previous text feature vector, a middle text feature vector, and a following text feature vector of the two data segments; and

   a step of producing a bipartite graph of the data segment and the data segment relationship, and inputting the feature vector of the data segment, the feature vector of the data segment relationship, and a connection relation of the two in the bipartite graph into a pre-trained graph convolutional network model to output a class label of the data segment and a class label of the data segment relationship.

2. The method in accordance with claim 1, further comprising:
   a step of training the boundary extraction model, including

   converting at least one training text into structured data, wherein, the structured data involves at least one examination time, examination item, examination result, and a correspondence of the three, and in the training text, the data segment and its class as well as relationships between the data segments and classes of these relationships have been marked in advance; and training the boundary extraction model by utilizing the training text, wherein, an input of the boundary extraction model is the examination result, an output of the boundary extraction model is an extraction result of all the data segments in the training text, and the data segments involve at least one attribute and entity in the training text.

3. The method in accordance with claim 2, wherein, a training goal for training the boundary extraction model is to minimize a loss function $L_{span}$ that is calculated by adopting

$$P(\hat{t_i}|s) = Softmax(W_{span}h_i)$$

and

$$L_{span} = -\frac{1}{|s|}\sum_{i=1}^{|s|} logP(\hat{t_i} = t_i|s),$$

here, $\hat{t_i}$ is an i-th label predicted by the boundary extraction model in the examination result; s is the input examination result; $W_{span}$ is a weight parameter of the boundary extraction model that is inclusive of a bi-directional long short-term memory network and a classifier; $h_i$ is a word feature vector representation output from the bi-directional long short-term memory network; $|s|$ is a length of the examination result; and $t_i$ is a real label of an i-th word in the examination result.

**4.** The method in accordance with claim 3, further comprising:
a step of training the graph convolutional network model, including

converting a word feature vector of the data segment in the training text into a text feature vector, performing positional encoding on the data segment to generate a position feature vector, and connecting the text feature vector and the position feature vector to form a feature vector of the data segment;
generating a feature vector of the data segment relationship between any one entity and attribute in the training text, wherein, the feature vector of the data segment relationship involves the feature vectors of two data segments as well as a previous text feature vector, a middle text feature vector, and a following text feature vector of the two data segments; and
producing a bipartite graph by using the data segment and the data segment relationship in the training text, and training the graph convolutional network model by utilizing the feature vector of the data segment and the feature vector of the data segment relationship in the training text, wherein, an input of the graph convolutional network model is the feature vector of the data segment, the feature vector of the data segment relationship, and a connection relation of the two in the bipartite graph, and an output of the graph convolutional network model is a class label of the data segment and a class label of the data segment relationship.

**5.** The method in accordance with claim 4, wherein,
a training goal for training a joint extraction model consisting of the graph convolutional network model and the boundary extraction model is to minimize a loss function L that is calculated by adopting

$$L = L_{span} + L_{ea} + L_{rel},$$

$$P(\hat{y}|e_i, s) = Softmax(W_{ea}F_{e_i}),$$

$$L_{ea} = -\frac{1}{|\hat{\varepsilon}|}\Sigma_{e_i \in \hat{\varepsilon}} logP(\hat{y} = y|e_i, s),$$

$$P(\hat{l}|r_{ij}, s) = Softmax(W_{rel}F_{r_{ij}}), \text{ and}$$

$$L_{rel} = -\Sigma_{r_{ij}} \frac{logP(\hat{l}=l|r_{ij}, s)}{|r_{ij}|},$$

here, $L_{ea}$ is a loss function of a data segment class in the graph convolutional network model; $L_{rel}$ is a loss function of a data segment relationship class in the graph convolutional network model; $\hat{y}$ is a class label of an i-th data segment predicted by the graph convolutional network model; $e_i$ is the i-th data segment; s is the input examination result; $W_{ea}$ and $W_{rel}$ are weight parameters of the graph convolutional network model; $F_{e_i}$ is a feature vector representation of the i-th data segment output from the graph convolutional network model; $|\hat{\varepsilon}|$ is a total number of all the data segments; $t_i$ is a real label of the i-th data segment; $\hat{l}$ is a class label of a relationship $r_{ij}$ predicted by the graph convolutional network model; the relationship $r_{ij}$ is a relationship between a data segment i and a data segment j; $F_{r_{ij}}$ is a feature vector representation of the relationship $r_{ij}$ output from the graph convolutional network model; $|r_{ij}|$ is a total number of all the relationships; and l is a real label of the relationship $|r_{ij}|$.

**6.** The method in accordance with claim 1, wherein, the step of converting a text to be processed into structured data includes

extracting at least one examination item in the text to be processed by using a dictionary of examination item terms;
extracting at least one examination time in the text to be processed by utilizing a time extraction rule and template;
extracting, based on at least one text punctuation mark and text distance, at least one examination result in the text to be processed; and
establishing a mapping relation of the examination item, the examination time, and the examination result.

**7.** An apparatus for joint-extracting clinical text entities and attributes as well as their relationships, comprising:

a conversion module configured to convert a text to be processed into structured data, wherein, the structured data includes at least one examination result;
a boundary extraction module configured to input the examination result into a pre-trained boundary extraction model to output an extraction result of all data segments in the text to be processed, wherein, the data segments include at least one attribute and entity in the text to be processed;
a feature vector generation module configured to convert a word feature vector of the data segment into a text feature vector, perform positional encoding on the data segment to generate a position feature vector, and connect the text feature vector and the position feature vector to form a feature vector of the data segment, and configured to generate a feature vector of a data segment relationship between any one entity and attribute, wherein, the feature vector of the data segment relationship includes the feature vectors of two data segments as well as a previous text feature vector, a middle text feature vector, and a following text feature vector of the two data segments; and

a processing module configured to produce a bipartite graph of the data segment and the data segment relationship, and input the feature vector of the data segment, the feature vector of the data segment relationship, and a connection relation of the two in the bipartite graph into a pretrained graph convolutional network model to output a class label of the data segment and a class label of the data segment relationship.

8. The apparatus in accordance with claim 7, further comprising:
a first training module configured to train the boundary extraction model, including

a conversion unit configured to convert at least one training text into structured data, wherein, the structured data involves at least one examination time, examination item, examination result, and a correspondence of the three, and in the training text, the data segment and its class as well as relationships between the data segments and classes of these relationships have been marked in advance; and
a first training unit configured to train the boundary extraction model by utilizing the training text, wherein, an input of the boundary extraction model is the examination result, an output of the boundary extraction model is an extraction result of all the data segments in the training text, and the data segments involve at least one attribute and entity in the training text.

9. The apparatus in accordance with claim 8, wherein, a training goal for training the boundary extraction model is to minimize a loss function $L_{span}$ that is calculated by adopting

$$P(\hat{t_i}|s) = \text{Softmax}(W_{span}h_i)$$

and

$$L_{span} = -\frac{1}{|s|}\sum_{i=1}^{|s|}\log P(\hat{t_i} = t_i|s),$$

here, $\hat{t_i}$ is an i-th label predicted by the boundary extraction model in the examination result; s is the input examination result; $W_{span}$ is a weight parameter of the boundary extraction model that is inclusive of a bi-directional long short-term memory network and a classifier; $h_i$ is a word feature vector representation output from the bi-directional long short-term memory network; |s| is a length of the examination result; and $t_i$ is a real label of an i-th word in the examination result.

10. The apparatus in accordance with claim 9, further comprising:

a second training module configured to train the graph convolutional network model, including
a feature vector generation unit configured to convert a word feature vector of the data segment in the training text into a text feature vector, perform positional encoding on the data segment to generate a position feature vector, and connect the text feature vector and the position feature vector to form a feature vector of the data segment, and configured to generate a feature vector of the data segment relationship between any one entity and attribute in the training text, wherein, the feature vector of the data segment relationship involves the feature vectors of two data segments as well as a previous text feature vector, a middle text feature vector, and a following text feature vector of the two data segments; and
a second training unit configured to produce a bipartite graph by using the data segment and the data segment relationship in the training text, and train the graph convolutional network model by utilizing the feature vector of the data segment and the feature vector of the data segment relationship in the training text, wherein, an input of the graph convolutional network model is the feature vector of the data segment, the feature vector of the data segment relationship, and a connection relation of the two in the bipartite graph, and an output of the graph convolutional network model is a class label of the data segment and a class label of the data segment relationship.

11. The apparatus in accordance with claim 10, wherein, a training goal for training a joint extraction model consisting of the graph convolutional network model and the boundary extraction model is to minimize a loss function L that is calculated by adopting

$$L = L_{span} + L_{ea} + L_{rel},$$

$$P(\hat{y}|e_i, s) = \text{Softmax}(W_{ea}F_{e_i}),$$

$$L_{ea} = -\frac{1}{|\hat{\varepsilon}|}\sum_{e_i \in \hat{\varepsilon}} \log P(\hat{y} = y|e_i, s),$$

$$P(\hat{l}|r_{ij}, s) = \text{Softmax}(W_{rel}F_{r_{ij}}), \text{ and}$$

$$L_{rel} = -\sum_{r_{ij}} \frac{\log P(\hat{l}=l|r_{ij},s)}{|r_{ij}|},$$

here, $L_{ea}$ is a loss function of a data segment class in the graph convolutional network model; $L_{rel}$ is a loss function of a data segment relationship class in the graph convolutional network model; $\hat{y}$ is a class label of an i-th data segment predicted by the graph convolutional network model; $e_i$ is the i-th data segment; s is the input examination result; $W_{ea}$ and $W_{rel}$ are weight parameters of the graph convolutional network model; $F_{e_i}$ is a feature vector representation of the i-th data segment output from the graph convolutional network model; $|\hat{\varepsilon}|$ is a total number of all the data segments; $t_i$ is a real label of the i-th data segment; $\hat{l}$ is a class label of a relationship $r_{ij}$ predicted by the graph convolutional network model; the relationship $r_{ij}$ is a relationship between a data segment i and a data segment j; $F_{r_{ij}}$ is a feature vector representation of the relationship $r_{ij}$ output from the graph convolutional network model; $|r_{ij}|$ is a total number of all the relationships; and l is a real label of the relationship $|r_{ij}|$.

12. The apparatus in accordance with claim 7, wherein, the conversion module includes

a first extraction unit configured to extract at least one examination item in the text to be processed by using a dictionary of examination item terms;
a second extraction unit configured to extract at least one examination time in the text to be processed by utilizing a time extraction rule and template;
a third extraction unit configured to extract, based on at least one text punctuation mark and text distance, at least one examination result in the text to be processed; and
a mapping relation establishment unit configured to establish a mapping relation of the examination item, the examination time, and the examination result.

13. A non-transitory computer-readable medium storing computer-executable instructions for execution by a processor, wherein,

the computer-executable instructions, when executed, cause the processor to perform the method in accordance with any one of claims 1 to 6.

14. An apparatus for joint-extracting clinical text entities and attributes as well as their relationships, comprising:

a storage storing computer-executable instructions; and
a processor coupled to the storage,
wherein,
the computer-executable instructions, when executed, cause the processor to conduct the method in accordance with any one of claims 1 to 6.

15. A computer program for causing a computer to execute the method in accordance with any one of claims 1 to 6.

# FIG.1

START

_S101

CONVERT TEXT TO BE PROCESSED INTO STRUCTURED DATA THAT CONTAINS AT LEAST ONE EXAMINATION RESULT

_S102

INPUT EXAMINATION RESULT INTO PRE-TRAINED BOUNDARY EXTRACTION MODEL TO OUTPUT EXTRACTION RESULT OF ALL DATA SEGMENTS IN TEXT TO BE PROCESSED, DATA SEGMENTS INCLUDING AT LEAST ONE ATTRIBUTE AND ENTITY IN TEXT TO BE PROCESSED

_S103

CONVERT WORD FEATURE VECTOR OF DATA SEGMENT INTO TEXT FEATURE VECTOR, PERFORM POSITIONAL ENCODING ON DATA SEGMENT TO GENERATE POSITION FEATURE VECTOR, AND CONNECT TEXT FEATURE VECTOR AND POSITION FEATURE VECTOR TO FORM FEATURE VECTOR OF DATA SEGMENT; AND GENERATE FEATURE VECTOR OF DATA SEGMENT RELATIONSHIP BETWEEN ANY ONE ENTITY AND ATTRIBUTE, FEATURE VECTOR OF DATA SEGMENT RELATIONSHIP CONTAINING FEATURE VECTORS OF TWO DATA SEGMENTS AS WELL AS PREVIOUS TEXT FEATURE VECTOR, MIDDLE TEXT FEATURE VECTOR, AND FOLLOWING TEXT FEATURE VECTOR OF THE TWO DATA SEGMENTS

_S104

PRODUCE BIPARTITE GRAPH BY USING DATA SEGMENT AND DATA SEGMENT RELATIONSHIP, AND INPUT FEATURE VECTOR OF DATA SEGMENT, FEATURE VECTOR OF DATA SEGMENT RELATIONSHIP, AND CONNECTION RELATION OF THE TWO IN BIPARTITE GRAPH INTO PRE-TRAINED GRAPH CONVOLUTIONAL NETWORK MODEL, SO AS TO OUTPUT CLASS LABEL OF DATA SEGMENT AND CLASS LABEL OF DATA SEGMENT RELATIONSHIP

STOP

# FIG.2

辅助检查: 2019-05-08TCT:慢性宫颈炎（中度），部分上皮细胞非典型增生，倾向低级别上皮内病变。（菏泽市中医院）2019-05-11HPV16+。（菏泽市中医院）2019-05-14阴道镜活检病理示:（宫颈2、4、6、9、12点）慢性宫颈炎伴腺体鳞化，鳞状上皮细胞乳头状增生，部分上皮细胞轻度非典型增生，呈低级别上皮内病变，CINⅠ级。（菏泽市中医院）2019-05-17我院病理会诊意见:（宫颈2、4、6、9、12点）慢性宫颈炎伴腺体鳞状化生，其中2点和12点处部分鳞状上皮呈高级别上皮内病变（CINⅡ级）。

# FIG.3

```
[
    {
        "EXAMINATION ITEM": "TCT",
        "EXAMINATION TIME": "2019-05-08",
        "EXAMINATION RESULT": "慢性宫颈炎（中度），部分上皮细胞非典型增生，倾向低级别上皮内病变。"
    },
    {
        "EXAMINATION ITEM": "阴道镜活检病理",
        "EXAMINATION TIME": "2019-05-14",
        "EXAMINATION RESULT": "（宫颈2、4、6、9、12点）慢性宫颈炎伴腺体鳞化，鳞状上皮细胞乳头状增生，部分上皮细胞轻度非典型增生，呈低级别上皮内病变，CINⅠ级。"
    },
    {
        "EXAMINATION ITEM": "病理会诊意见",
        "EXAMINATION TIME": "2019-05-17",
        "EXAMINATION RESULT": "（宫颈2、4、6、9、12点）慢性宫颈炎伴腺体鳞状化生，其中2点和12点处部分鳞状上皮呈高级别上皮内病变（CINⅡ级）"
    }
]
```

# FIG.4

OUTPUT: B-A I-A I-A I-A I-A I-A B-A I-A I-A B-E I-E O O O

softmax

BiLSTM

EMBEDDING REPRESENTATION

INPUT: ... 部 分 上 皮 细 胞 非 典 型 增 生 ， 倾 向 ...

B F S P X B F D X Z S , Q X

EP 4 068 149 A1

# FIG.5

EP 4 068 149 A1

# FIG.6

SPAN POSITION
EMBEDDING

SPAN TEXT
EMBEDDING

$$h_i = s_i + p_i$$

DIMENSIONAL
TRANSFORMATION
MODEL

| MLP | MLP | MLP |
| pooling | pooling | pooling |
| CNN | CNN | CNN |

SPAN SEARCH
RESULT

... 部 分 上 皮 细 胞 | 非 典 型 | 增 生 ， 倾 向 ...

B F S P X B F D X Z S ， Q X

a1 a2 e3

EP 4 068 149 A1

FIG.7

SPAN RELATIONSHIP FEATURE

$r_{13}$

SIZE TRANSFORMATION

MLP

FEATURE CONNECTION

CONNECTION

SPAN POSITION FEATURE

$h_1$     $h_3$

SPAN TEXT FEATURE

PREVIOUS TEXT FEATURE          MIDDLE TEXT FEATURE          FOLLOWING TEXT FEATURE

DIMENSIONAL TRANSFORMATION MODEL

MLP    MLP    MLP    MLP    MLP

pooling  pooling  pooling  pooling  pooling

CNN    CNN    CNN    CNN    CNN

SPAN SEARCH RESULT

... 部 分 上 皮 细 胞 非 典 型 增 生 ， 倾 向 ...

B F S P X B F D X Z S , Q X

a1          a2      e3

EP 4 068 149 A1

# FIG.8

| 21 | 22 | 23 | 24 |
|----|----|----|----|
| CONVERSION MODULE | BOUNDARY EXTRACTION MODULE | FEATURE VECTOR GENERATION MODULE | PROCESSING MODULE |

# FIG.9

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DUAN GUIDUO ET AL: "A Relational Adaptive Neural Model for Joint Entity and Relation Extraction", FRONTIERS IN NEUROROBOTICS, vol. 15, 16 March 2021 (2021-03-16), page 635492, XP055952929, DOI: 10.3389/fnbot.2021.635492 Retrieved from the Internet: URL:https://europepmc.org/backend/ptpmcren der.fcgi?accid=PMC8008121&blobtype=pdf> * the whole document * | 1-15 | INV. G06F40/279 G06F40/53 G16H50/00 G06N3/02 G06F40/295 |
| X | CN 112 163 416 A (BEIJING INSTITUTE TECH) 1 January 2021 (2021-01-01) * the whole document * | 1-15 | |
| X | XI QISEN ET AL: "Chinese Named Entity Recognition: Applications and Challenges", 7 March 2021 (2021-03-07), ARXIV.ORG, PAGE(S) 51 - 81, XP047578589, * the whole document * | 1-15 | |
| | | | TECHNICAL FIELDS SEARCHED (IPC) |
| X | WO 2021/030915 A1 (GOVERNING COUNCIL UNIV TORONTO [CA]; UNITY HEALTH TORONTO [CA]) 25 February 2021 (2021-02-25) * the whole document * | 1-15 | G06F G16H G06N |
| X | CN 109 800 411 A (HARBIN INST TECHNOLOGY SHENZHEN) 24 May 2019 (2019-05-24) * the whole document * | 1-15 | |
| A | WO 2019/137562 A2 (ALIBABA GROUP HOLDING LTD [CN]) 18 July 2019 (2019-07-18) * the whole document * | 1-15 | |

The present search report has been drawn up for all claims

| Place of search | Date of completion of the search | Examiner |
|---|---|---|
| Berlin | 19 August 2022 | Lechenne, Laurence |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 16 2334

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2022

| Patent document cited in search report | | Publication date | Patent family member(s) | | Publication date |
|---|---|---|---|---|---|
| CN 112163416 | A | 01-01-2021 | NONE | | |
| WO 2021030915 | A1 | 25-02-2021 | CA | 3148554 A1 | 25-02-2021 |
| | | | EP | 4018353 A1 | 29-06-2022 |
| | | | US | 2022172725 A1 | 02-06-2022 |
| | | | WO | 2021030915 A1 | 25-02-2021 |
| CN 109800411 | A | 24-05-2019 | NONE | | |
| WO 2019137562 | A2 | 18-07-2019 | AU | 2019207309 B2 | 01-10-2020 |
| | | | CA | 3061432 A1 | 18-07-2019 |
| | | | CN | 111295670 A | 16-06-2020 |
| | | | EP | 3646245 A2 | 06-05-2020 |
| | | | SG | 11201909950Q A | 28-11-2019 |
| | | | US | 10740561 B1 | 11-08-2020 |
| | | | WO | 2019137562 A2 | 18-07-2019 |

## REFERENCES CITED IN THE DESCRIPTION

**Patent documents cited in the description**

- CN 202110348806X